(19) Europäisches Patentamt
European Patent Office
Office européen des brevets

(11) Veröffentlichungsnummer: **0 000 476 B1**

# (12) EUROPÄISCHE PATENTSCHRIFT

(45) Veröffentlichungstag der Patentschrift: **07.10.81**

(51) Int. Cl.³: **C 07 D 313/00**

(21) Anmeldenummer: **78100237.3**

(22) Anmeldetag: **26.06.78**

## (54) Verfahren zur Herstellung von Cyclopentadecanolid.

(30) Priorität: **13.07.77 DE 2731543**

(43) Veröffentlichungstag der Anmeldung: **07.02.79 Patentblatt 79/3**

(45) Bekanntmachung des Hinweises auf die Patenterteilung: **07.10.81 Patentblatt 81/40**

(84) Benannte Vertragsstaaten: **BE CH DE FR GB NL**

(56) Entgegenhaltungen:
**DE-A-2 410-859**
**THE JOURNAL OF THE AMERICAN CHEMICAL SOCIETY, vol. 58, Jan.-Juni 1936; Seiten 654-56.**

**THE CHEMISTRY OF OPEN-CHAIN ORGANIC NITROGEN COMPOUNDS, volume II, Benjamin, New York 1966 P.A.S. SMITH, Seite 178**

**JOURNAL OF HETEROCYCLIC CHEMISTRY, vol. 6 (3), Juni 1969, Seiten 349-60**

(73) Patentinhaber: **HAARMANN & REIMER GMBH, Postfach 138, D-3450 Holzminden (DE)**

(72) Erfinder: **Bauer, Kurt, Dr., Corveyblick 41, D-3450 Holzminden (DE)**
Erfinder: **Körber, Alfred, Dr., Bismarckstrasse 4, D-3450 Holzminden (DE)**

(74) Vertreter: **Dill, Erwin, Dr., c/o BAYER AG Zentralbereich Patente Marken und Lizenzen Bayerwerk, D-5090 Leverkusen 1 (DE)**

(56) Entgegenhaltungen:
**JOURNAL OF THE CHEMICAL SOCIETY, CHEMICAL COMMUNICATIONS, 1972, Seite 1078 (Oktober 1972) MAHAJAN et al.: "Synthesis of oximino-macrolides"**

**CHEMICAL ABSTRACTS, vol. 86, Seite 461 (1977) 5336z ZAKHARKIN et al.: "Cyclopentadecanolides"**

**CHEMICAL ABSTRACTS, vol. 78, Seite 364 (1973) 158968s. YASUKAWA TADASHI: "Large ring lactones"**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG.

EP 0 000 476 B1

# Verfahren zur Herstellung von Cyclopentadecanolid

Die Erfindung betrifft ein Verfahren zur Herstellung von Cyclopentadecanolid.

Cyclopentadecanolid ist ein natürlicher Moschus-Riechstoff. Zu seiner Herstellung sind verschiedene Verfahren bekannt, die jedoch eine Reihe von Nachteilen aufweisen, insbesondere, dass sie lediglich geringe Ausbeuten ergeben, und dass die Ausgangsmaterialien nicht leicht zugänglich sind.

Von wirtschaftlichem Interesse sind lediglich zwei Verfahren. Bei dem in der DE-AS 2 026 056 beschriebenen Verfahren wird das gemäss DE-AS 2 136 496 aus Cyclododecanon leicht zugängliche 13-Oxa-bicyclo[10.4.0]-hexadecen-[1(12)] mit Wasserstoffperoxid oder einem Alkylhydroperoxid in ein Peroxid überführt, dieses entweder thermisch oder durch Bestrahlung mit UV-Licht in ein Gemisch aus Cyclopentadecanolid und Cyclopentadecenolid gespalten und das Gemisch hydriert. Das Verfahren hat den Nachteil, dass das Arbeiten mit Peroxiden besondere Vorsichtsmassnahmen erfordert und dass die Ausbeute an Cyclopentadecanolid nur höchstens 57,9%, bezogen auf 13-Oxa-bicyclo[10.4.0]-hexadecen-[1(12)], beträgt.

Bei dem zweiten Verfahren wird 15-Hydroxypentadecansäure durch Vakuumdestillation ihres Polyesters cyclisiert [J. Am. Chem. Soc. 58, 654 (1936)]. Dieses Verfahren hat den Nachteil, dass die Ausgangsmaterialien zur Herstellung der 15-Hydroxy-pentadecansäure, wie 9,10,16-Trihydroxy-hexadecancarbonsäure (J. Chem. Soc. 1963, 3505), Erucylalkohol (J.Chem. Soc. 1962, 2348), Undec-10-ensäure [Tetrahedron 19, 905 (1963); Japanische Patentanmeldung 68 04 262] für ein wirtschaftliches Verfahren zu teuer sind oder nicht in ausreichender Menge zur Verfügung stehen.

In dem russischen Erfinderschein 521 274 wird ein Verfahren beschrieben, bei dem 13-Oxa-bicyclo[10.4.0]-hexadecen-[1(12)] mit Butylnitrit/$NaHSO_3$ in das 12-Keto-cyclopentadecanolid übergeführt und dieses nach Clemmensen zum Cyclopentadecanolid reduziert wird. Die Ausbeute an Cyclopentadecanolid beträgt bei diesem Verfahren jedoch nur 36% wie aus den Beispielen hervorgeht. Ausserdem ist die für die Clemmensen-Reduktion aufgewendete Reaktionszeit von 90 Stunden technisch nicht tragbar.

Der Erfindung liegt daher die Aufgabe zugrunde, ein Verfahren zu entwickeln, das ausgehend vom leicht zugänglichen 13-Oxa-bicyclo[10.4.0]-hexadecen-[1(12)] in guten Ausbeuten und unter Vermeidung aufwendiger Verfahrensschritte Cyclopentadecanolid in hoher Reinheit liefert.

Diese Aufgabe wird erfindungsgemäss dadurch gelöst, dass man 13-Oxa-bicyclo[10.4.0]-hexadecen-[1(12)] in an sich bekannter Weise in ein Lacton der allgemeinen Formel I

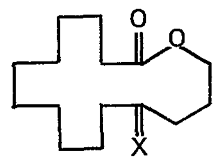

(I)

überführt, in der

X ein Sauerstoffatom, die Gruppe = NOH oder $=N\text{-}NH_2$ bedeutet,

das Lacton der allgemeinen Formel I unter Öffnung des Lactonringes zur 15-Hydroxypentadecansäure reduziert und diese in an sich bekannter Weise zum Cyclopentadecanolid cyclisiert.

Überraschenderweise werden bei dem erfindungsgemässen Verfahren, obwohl bei diesem eine Öffnung des Macrozyclus erfolgt, wesentlich bessere Ausbeuten erhalten als bei den bekannten Verfahren, die ebenfalls vom 13-Oxa-bicyclo[10.4.0]-hexadecen-[1(12)] ausgehen und bei denen der Macrozyclus in allen Reaktionsschritten erhalten bleibt. Mit Hilfe des erfindungsgemässen Verfahrens wird mit technisch ohne Aufwand durchführbaren Verfahrensschritten Cyclopentadecanolid hoher Reinheit in Ausbeuten von 75 bis 85% der Theorie, bezogen auf 13-Oxa-bicyclo[10.4.0]-hexadecen-[1(12)] erhalten.

Die Überführung des 13-Oxa-bicyclo[10.4.0]-hexadecen-[1(12)] in ein Lacton der allgemeinen Formel I, in der X für ein Sauerstoffatom steht, kann wie in der DE-OS 2 410 859 beschrieben durch Ozonolyse der Doppelbindung oder entsprechend J.Org.Chem. 37, 581 (1972) mit einem Überschuss einer Persäure erfolgen.

Zur Überführung des 13-Oxa-bicyclo[10.4.0]-hexadecen-[1(12)] in ein Lacton der allgemeinen Formel I, in der X für die Gruppe =NOH steht, hat es sich als vorteilhaft erwiesen, 13-Oxa-bicyclo[10.4.0]-hexadecen-[1(12)] gemäss J.C.S. Chem. Comm. 1972, 1078 mit Nitrosylchlorid oder mit unter den Reaktionsbedingungen Nitrosylionen bildenden Stickstoffverbindungen, wie Alkylnitriten oder Alkalinitriten, umzusetzen.

Das entstandene 12-Oximinocyclopentadecanolid kann anschliessend in an sich bekannter Weise, z.B. mit Natriumhydrogensulfit, zum 12-Ketocyclopentadecanolid, d.h. einer Verbindung der allgemeinen Formel I, in der X für das Sauerstoffatom steht, hydrolisiert werden oder mit Hydrazinhydrat in das entsprechende Hydrazon, d.h. eine Verbindung der allgemeinen Formel I, in der X für die $=NNH_2$-Gruppe steht, umgewandelt werden. Das Hydrazon lässt sich nach Wolff-Kishner mit Natrium oder einem Natriumalkoholat, das Keton nach Huang-Minlon mit Hydrazin in Gegenwart von Natrium- oder Kaliumhydroxid unter gleichzeitiger Spaltung des Lactonringes zur 15-Hydroxypentadecansäure reduzieren.

In einer besonders bevorzugten Ausführungsform des erfindungsgemässen Verfahrens wird jedoch das 12-Oximino-cyclopentadecanolid unmittelbar, d.h. ohne es zum Keton zu verseifen,

mit einem Überschuss an Hydrazinhydrat in Gegenwart von Alkalihydroxid unter Spaltung des Lactonringes zur 15-Hydroxypentadecansäure reduziert. Die reduzierende Spaltung erfolgt durch Erwärmen der Mischung von Oxim, Hydrazin und Alkalihydroxid in einem höher siedenden organischen Lösungsmittel, wie Äthylen- oder Diäthylenglykol, auf Temperaturen von 150 bis 200°C.

Das Molverhältnis Hydrazin:12-Oximinocyclopentadecanolid sollte zwischen 1,5–3:1 liegen. Hydrazin wird vorzugsweise in Form von 80%igem Hydrazinhydrat eingesetzt. Als Alkalihydroxid werden vorzugsweise Natrium- oder Kaliumhydroxid verwendet. Das Molverhältnis Alkalihydroxid: 12-Oximinocyclopentadecanolid beträgt vorteilhaft 3–5:1.

Grundsätzlich ist es möglich, ausser Oxim und Hydrazon auch andere Azomethinderivate des 12-Ketocyclopentadecanolids, z.B. das Semicarbazon oder substituiertes Phenylhydrazone nach Wolff-Kishner oder Huang-Minlon zur 15-Hydroxypentadecansäure zu reduzieren. Diese Derivate bieten jedoch keine Vorteile gegenüber Oxim oder Hydrazon, sondern erfordern lediglich einen zusätzlichen Verfahrensschritt.

Die Cyclisierung der 15-Hydroxypentadecansäure kann nach den üblichen, aus der Literatur bekannten Verfahren zur Herstellung von Lactonen aus Hydroxycarbonsäuren erfolgen. Als besonders vorteilhaft hat sich die vorstehend genannte Methode von Spanagel und Carothers [J. Am. Chem. Soc. 58, 654 (1936)] erwiesen, bei der zunächst aus 15-Hydroxypentadecansäure ein linearer Polyester hergestellt wird, der dann in Gegenwart eines Depolymerisationskatalysators, wie Magnesiumchlorid, Magnesiumoxid, Bleidioxid usw. depolymerisiert wird.

Da in dem erfindungsgemässen Verfahren praktisch alle Zwischenprodukte und das Endprodukt kristallin anfallen, ist eine Reinigung auf jeder Stufe möglich. Auf diese Weise wird das Endprodukt in hoher Reinheit erhalten und eine spezielle Reinigung entfällt.

Beispiel 1

Die Mischung von 221 g (1,85 Mol) Isoamylnitrit, 378 g (1,7 Mol) 13-Oxa-bicyclo[10.4.0]-hexadecen-[1(12)], 850 ml Äthanol und 850 ml Wasser wurde auf −5°C abgekühlt und innerhalb von 75 Minuten tropfenweise mit 129 ml 10%iger Salzsäure versetzt. Die Reaktionsmischung wurde 2 Stunden bei 0 bis +5°C gerührt, anschliessend mit 1000 ml Wasser versetzt. Das ausgefallene Kristallisat wurde abgetrennt, neutral gewaschen und getrocknet.

Ausbeute: 448,3 g (= 97% der Theorie) 12-Oximinocyclopentadecanolid

Beispiel 2

Die Mischung von 379,5 g (5,5 Mol) Natriumnitrit, 1110 g (5 Mol) 13-Oxa-bicyclo[10.4.0]-hexadecen-[1(12)], 2520 ml Isopropanol und 2520 ml Wasser wurde auf eine Temperatur zwischen 0 bis +5°C abgekühlt und in diesem Temperaturbereich innerhalb von 2 Stunden tropfenweise mit 420 g 38%iger Salzsäure versetzt. Anschliessend wurden 138 g 38%ige Salzsäure innerhalb von 20 Minuten unter besonders intensiver Kühlung zugegeben. Die Reaktionsmischung wurde 2 Stunden bei 0 bis +5°C gerührt, anschliessend innerhalb von 2,5 Stunden mit Eiswasser versetzt. Das ausgefallene Kristallisat wurde abgetrennt, neutral gewaschen und getrocknet.

Ausbeute: 1299 g (= 95,7% der Theorie) 12-Oximinocyclopentadecanolid

Beispiel 3

In einem mit Innenthermometer, Rückflusskühler und Rührer versehenen 4-l-Dreihalskolben wurden 1000 ml Diäthylenglykol vorgelegt. Anschliessend wurden unter Kühlen nacheinander 280 g (5 Mol) Kaliumhydroxid, 269 g (1 Mol) 12-Oximinocyclopentadecanolid und 187,5 g 80%iges Hydrazinhydrat zugegeben. Dann wurde die Apparatur mit Stickstoff gespült und das Reaktionsgemisch 2 Stunden auf Rückflusstemperatur erhitzt. Danach wurde das überschüssige Hydrazinhydrat im Gemisch mit Wasser abdestilliert. Dabei wurde die Sumpftemperatur auf 185 bis 195°C gesteigert. Diese Temperatur wurde solange gehalten, bis die Stickstoffentwicklung beendet war (etwa 9 Stunden). Das Reaktionsgemisch wurde mit einem Liter Eiswasser versetzt und mit Salzsäure auf einen pH-Wert von 2 angesäuert. Das ausgefallene Reaktionsprodukt wurde in der Wärme mit Essigester extrahiert. Beim Abkühlen der warmen Essigesterlösung kristallisierte das Produkt wieder aus. Das Produkt wird abgetrennt und getrocknet.

Ausbeute: 239 g (= 92,6% der Theorie) 15-Hydroxypentadecansäure:

(Schmelzpunkt: 84°C).

Bei Verwendung von jeweils 5 Mol Lithiumhydroxid, Natriumhydroxid oder Natriumäthylat anstelle von 5 Mol Kaliumhydroxid betrugen die Ausbeuten an 15-Hydroxypentadecansäure 87 bis 91,6% der Theorie.

Beispiel 4

403 g (1,5 Mol) 12-Oximinocyclopentadecanolid (hergestellt gemäss Beispiel) wurden in einem Gemisch aus 2 l Äthanol und 2 l 40%iger Natriumbisulfitlösung 2,5 Stunden auf Rückflusstemperatur erhitzt. Dann wurde die Reaktionsmischung mit 3 l Wasser versetzt und mit Äther extrahiert. Die organische Phase wurde abgetrennt, neutral gewaschen und getrocknet. Anschliessend wurde das Lösungsmittel abdestilliert und der Rückstand im Vakuum destilliert (Kp: 150–153°C/1,73 Pa). Ausbeute: 332 g (= 87% der Theorie) 12-Keto-cyclopentadecanolid.

Beispiel 5

50 g 12-Keto-cyclopentadecanolid (hergestellt gemäss Beispiel 4) wurden mit 36,6 g 80%igem Hydrazinhydrat und 54,7 g Kaliumhydroxid in 150 ml Diäthylenglykol unter den in Beispiel 3 beschriebenen Reaktionsbedingungen umgesetzt.
Ausbeute: 48 g (94,4% der Theorie) 15-Hydroxypentadecansäure.

Beispiel 6

385 g (1,49 Mol) 15-Hydroxypentadecansäure wurden in einer Destillationsapparatur 3 Stunden auf 160°C erhitzt. Dann wurde Wasserstrahlvakuum angelegt, um das Restwasser zu entfernen. Der Rückstand wurde mit 23 g Magnesiumchlorid gemischt und unter Rühren und einem Vakuum von 66,5 Pa in eine auf 280°C vorgeheizte Destillationsapparatur getropft. Bei 140°C/66,5 Pa gingen 330 g (93,2% der Theorie, bezogen auf eingesetzte 15-Hydroxypentadecansäure) Cyclopentadecanolid über.

**Patentansprüche**

1) Verfahren zur Herstellung von Cyclopentadecanolid, dadurch gekennzeichnet, dass man 13-Oxy-bicyclo[10.4.0]-hexadecen-[1(12)] in an sich bekannter Weise in ein Lacton der allgemeinen Formel I

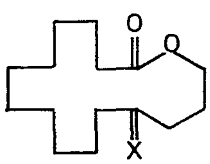

(I)

in der
X ein Sauerstoffatom, die Gruppe =NOH oder =N-$NH_2$ bedeutet,
überführt, das Lacton der allgemeinen Formel I unter Öffnung des Lactonringes zur 15-Hydroxydecansäure nach Wolff-Kishner bzw. Huang Minlon reduziert und diese in an sich bekannter Weise zum Cyclopentadecanolid cyclisiert.

2) Verfahren gemäss Anspruch 1, dadurch gekennzeichnet, dass man 13-Oxa-bicyclo[10.4.0]-hexadecen-[1(12)] mit Nitrosylchlorid oder einer unter den Reaktionsbedingungen Nitrosyl-Ionen bildenden Stickstoffverbindung zum 12-Oximinocyclopentadecanolid umsetzt, dieses mit Hydrazinhydrat nach Huang Minlon in Gegenwart von Alkalihydroxid in die 15-Hydroxypentadecansäure überführt, diese polymerisiert und den gebildeten linearen Polyester in Gegenwart von Depolymerisationskatalysatoren depolymerisiert.

**Claims**

1) Process for the preparation of cyclopentadecanolide, characterised in that 13-oxa-bicyclo[10.4.0]-hexadecene-[1(12)] is converted in a manner which is in itself known into a lactone of the general formula I

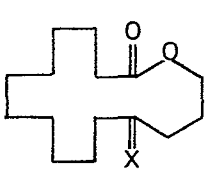

(I)

in which
X denotes an oxygen atom or the group =NOH or =N-$NH_2$,
the lactone of the general formula I is reduced, according to Wolff-Kishner or Huang Minlon, with opening of the lactone ring, to give 15-hydroxydecanoic acid and the latter is cyclised in a manner which is in itself known to give cyclopentadecanolide.

2) Process according to claim 1, characterised in that 13-oxa-bicyclo[10.4.0]-hexadecene-[1(12)] is reacted with nitrosyl chloride or a nitrogen compound which forms nitrosyl ions under the reaction conditions, to give 12-oximinocyclopentadecanolide, the latter is converted by means of hydrazine hydrate, according to Huang Minlon, in the presence of alkali metal hydroxide to 15-hydroxypentadecanoic acid, this acid is polymerised and the resulting linear polyester is depolymerised in the presence of depolymerisation catalysts.

**Revendications**

1. Procédé de préparation de cyclopentadécanolide, caractérisé en ce qu'on transforme, de façon connue en soi, le 13-oxa-bicyclo[10.4.0]-hexadécène-[1(12)] en une lactone de formule générale I:

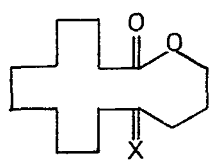

(I)

dans laquelle
X représente un atome d'oxygène, le groupe =NOH ou le groupe =N–$NH_2$,
on réduit cette lactone de formule générale I en acide 15-hydroxydécanoïque avec ouverture du noyau lactone suivant Wolff-Kishner ou Huang-Minlon, puis on soumet cet acide 15-hydroxydécanoïque à une cyclisation en cyclopentadécanolide de façon connue en soi.

2. Procédé suivant la revendication 1, caractérisé en ce qu'on fait réagir le 13-oxa-bicyclo[10.4.0]-hexadécène-[1(12)] avec du chlorure de nitrosyle ou un composé azoté formant des ions nitrosyle dans les conditions réactionnelles, pour obtenir le 12-oximinocyclopentadécanolide que l'on transforme, avec de l'hydrate d'hydrazine, suivant Huang-Minlon, en présence d'un hydroxyde alcalin, en acide 15-hydroxypentadécanoïque, puis on polymérise ce dernier et on dépolymérise le polyester linéaire formé en présence de catalyseurs de dépolymérisation.